# EUROPEAN PATENT SPECIFICATION

(11) **EP 1 776 365 B1**
(45) Date of publication and mention of the grant of the patent: **03.08.2011**
(21) Application number: 05753188.1
(22) Date of filing: 01.06.2005
(51) Int. Cl.: C07D 477/20

(54) **MEROPENEM INTERMEDIATE IN CRYSTALLINE FORM**
MEROPENEM-ZWISCHENPRODUKT IN KRISTALLINER FORM
INTERMEDIAIRE DE MEROPENEM SOUS FORME CRISTALLINE

(30) Priority: 02.06.2004 AT 9442004
(43) Date of publication of application: 25.04.2007
(73) Proprietor: SANDOZ AG, 4056 Basel (CH)
(72) Inventor: NADENIK, Peter, A-6300 Wörgl (AT); STORM, Ole, A-6330 Kufstein (AT); KREMMINGER, Peter, A-6330 Kufstein (AT)
(74) Representative: Wichmann, Hendrik
(86) International application number: PCT/EP2005/005909
(87) International publication number: WO 2005/118586

(56) References cited:
- EP-A- 0 188 816
- US-A- 4 933 333
- US-A- 5 541 317
- SUNAGAWA, MAKOTO ET AL: "Synthetic studies of carbapenem and penem antibiotics. V. Efficient synthesis of the 1.beta.-methylcarbapenem skeleton" CHEMICAL & PHARMACEUTICAL BULLETIN , 42(7), 1381-7 CODEN: CPBTAL; ISSN: 0009-2363, 1994, XP001207067
- MATSUMURA H ET AL: HETEROCYCLES, ELSEVIER SCIENCE PUBLISHERS B.V. AMSTERDAM, NL, vol. 41, no. 1, 1995, pages 147-159, XP002076865 ISSN: 0385-5414
- SUNAGAWA, MAKOTO ET AL: "A novel carbapenem antibiotic, SM-7338 structure-activity relationships" JOURNAL OF ANTIBIOTICS , 43(5), 519-32 CODEN: JANTAJ; ISSN: 0021-8820, 1990, XP002912928

## Description

The present invention relates to (4*R*,5*S*,6*S*)-(*p*-nitrobenzyl) 3-[[(3*S*,5*S*)-1(-*p-*nitrobenzyloxycarbonyl)-5-(dimethylaminocarbonyl)-3-pyrrolidinyl]thio]-6-[(1*R*)-1-hydroxyethyl]-4-methyl-7-oxo-1-azabicyclo[3.2.0]hept-2-ene-2-carboxylate in crystalline form (hereinafter also referred to as "crystalline compound 1") of formula 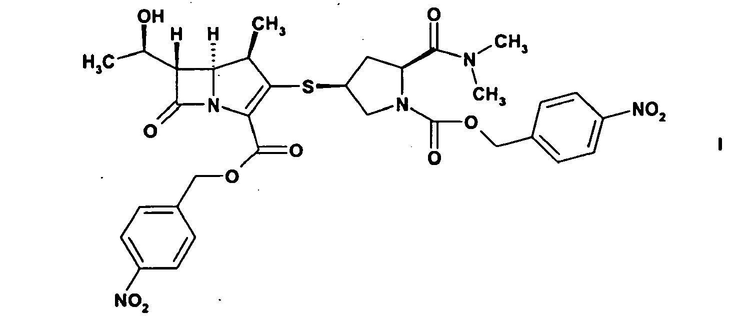 as well as processes for the production thereof.

The compound of formula I is an important known intermediate for the production of (4*R*,5*S*,6*S*)-3-[[(3*S*,5*S*)5-(dimethylaminocarbonyl)-3-pyrrolidinyl]thio]-6-[(1*R*)-1-hydroxyethyl]-4-methyl-7-oxo-1-azabicyclo[3.2.0]hept-2-ene-2-carboxylic acid (meropenem), a parenteral carbapenem having outstanding antibacterial properties. The compound of formula I in non-crystalline form, e.g. in amorphous form, in the form of an oil or in the form of a foamy or vitreous solid, is known from e.g. EP-A-0 188 816 and Sunagawa et al., Chem. Pharm. Bull., 42(7), 1381-7, 1994. Compared with crystalline forms of a compound, amorphous forms generally have considerably poorer material properties, such as stability, isolating properties or purity. In the multi-stage synthesis of a product which is to be obtained in sufficiently high purity, it can therefore be an enormous advantage to use crystalline forms of intermediate compounds instead of amorphous forms. A crystallisation step normally leads to a high purifying effect, and in addition, crystalline materials have substantially better stability than amorphous products.

The present invention is therefore based on the problem of providing a crystalline intermediate compound, which has the above-mentioned advantages and can be used in preparation processes for meropenem.

This problem is solved according to the invention by the compound of formula I in crystalline form. It has been found that, under certain conditions, the compound of formula I can be obtained in crystalline form.

Therefore, one aspect of this invention relates to the compound of formula I in crystalline form.

The physico-chemical properties of the compound of formula I in crystalline form may be described as follows:
1) External appearance of the crystals
   bacilli or short needles
2) Melting point
   156-159°C
3) X-ray powder diffractogram

The compound of formula I in crystalline form shows characteristic bands at the 2-theta diffraction angles of about 5.1, 12.8, 15.5, 17.6, 18.4, 21.1, 21.5 and 23.4.

The compound of formula 1 in crystalline may also be described by the x-ray powder diffractogram as shown in the following Table 1 and as depicted in figure 1.

**Table 1: X-ray powder diffractrogram of the compound of formula I in crystalline form; apparatus used: powder diffractometer AXS-BRUKER D-8, CuK-α radiation, Bragg-Brentano-Optics, scintillation counter, 40kV, 40mA, 0.01° steps, time 2 seconds, cut-off: 40°, standard sample carrier; evaluation was carried out using the software DiffracPlus™.**

| angle 2-Theta ° | d value Angstrom | intensity % |
|---|---|---|
| 5.091 | 17.34318 | 18.9 |
| 12.815 | 6.90257 | 32.3 |
| 13.181 | 6.71159 | 20.2 |
| 15.010 | 5.89754 | 22.7 |
| 15.480 | 5.71953 | 29.3 |
| 15.848 | 5.58766 | 27.5 |
| 16.348 | 5.41781 | 19.7 |
| 17.608 | 5.03292 | 79.6 |
| 18.367 | 4.82644 | 100.0 |
| 19.758 | 4.48985 | 22.8 |
| 20.316 | 4.36776 | 20.1 |
| 20.602 | 4.30770 | 18.3 |
| 21.133 | 4.20055 | 36.7 |
| 21.477 | 4.13404 | 37.5 |
| 22.120 | 4.01542 | 28.0 |
| 22.404 | 3.96519 | 31.0 |
| 23.411 | 3.79678 | 33.6 |
| 25.872 | 3.44089 | 22.5 |
| 26.605 | 3.34785 | 24.4 |
| 27.545 | 3.23564 | 31.7 |

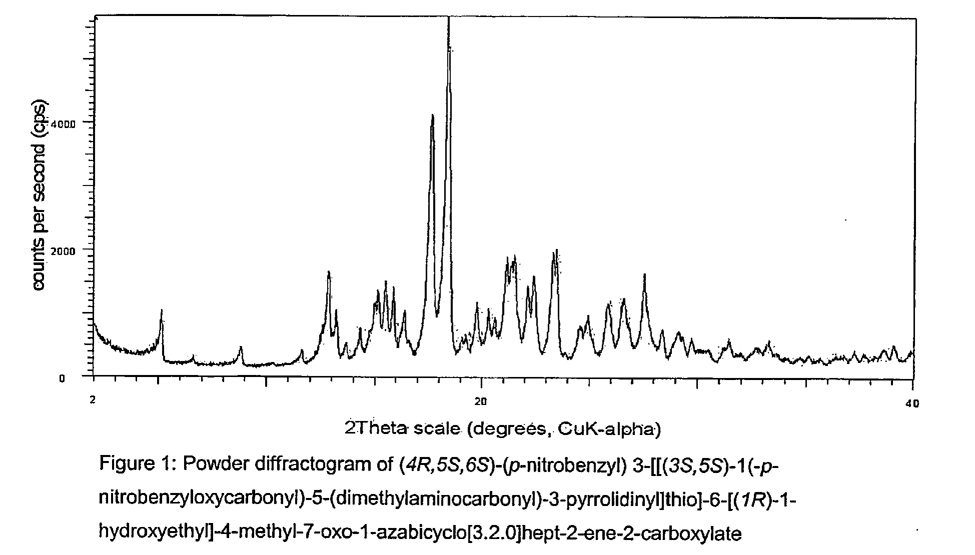

In a further aspect, the present invention relates to a process for the production of the compound of formula I in crystalline form.

The compound of formula I in crystalline form may be obtained whereby the compound of formula I in non-crystalline form is dissolved in an alkyl alkanoate, optionally also in a mixture with another organic solvent which is readily miscible with water. The solution of the compound of formula I may also be produced *in situ* by a coupling reaction between (2S,4S)-2-dimethylaminocarbonyl-4-mercapto-1-(*p*-nitrobenzyloxycarbonyl)-pyrrolidine or the salts thereof and a *p*-nitrobenzyl-(1*R*,5*R*,6S)-6-[(1*R*)-1-hydroxyethyl]-1-methylcarbapen-2-em-3-carboxylic acid ester which is activated at position 2 of the carbapenem frame, e.g. *p-*nitrobenzyl-(1*R*,5*R*,6*S*)-6-[(1*R*)-1-hydroxyethyl]-2-[(diphenylphosphono)oxy]-1-methylcarbapen-2-em-3-carboxylic acid ester, without intermediate isolation. Such coupling reactions are described, for example, in EP-A-126587.

If necessary, extraction with water follows, possibly even several times in succession, whereby the proportion of solvent miscible with water in the ethyl acetate phase continuously decreases.

The compound of formula I in crystalline form crystallises out of this alkyl alkanoate solution when the conditions necessary for crystallisation have been set. To set the conditions necessary for crystallisation, measures which allow crystallisation to set in and are known to a person skilled in the art, are appropriate, for example at least partly removing the solvent, such as by evaporating the solvent, optionally at reduced pressure, and/or by cooling the solvent, and/or by continually stirring. In addition, an anti-solvent may be added in order to complete crystallisation. Apolar hydrocarbons are especially suitable anti-solvents, e.g. aromatic hydrocarbons such as benzene, toluene, xylenes or alkanes, such as (C₆₋₁₂)-alkanes, e.g. (C₆₋₈)-alkanes, or cycloalkanes, such as (C₅-C₈)cycloalkanes. It is especially preferable to use heptane or cyclohexane, especially cyclohexane, as anti-solvents.

Alkyl alkanoates which are suitable as solvents are, for example, (C₁₋₆)alkyl esters of (C₂₋₄)-alkanoic acids. (C₁₋₆)alkyl esters of C₂-alkanoic acid (acetic acid) are preferred, especially (C₂₋₄)alkyl esters of acetic acid, such as ethyl acetate, isopropyl acetate, n-propyl acetate, n-butyl acetate, isobutyl acetate, sec.-butyl acetate or tert.-butyl acetate. Ethyl acetate, isopropyl acetate or n-butyl acetate are preferred in particular, especially ethyl acetate.

The crystalline compound of formula I which has crystallised out is isolated in the usual manner, e.g. filtered, washed and dried in a vacuum. The purity of the crystalline compound of formula I, which is obtained by the described process, is typically more than 98.5% (expressed as HPLC area percent), normally even more than 99.0% (HPLC area percent).

The yields are excellent and are normally at least 90% of theory, optionally up to 95% of theory.

A further aspect of this invention relates to the use of the compound of formula I in crystalline form, e.g. characterised by the powder diffractogram according to fig. 1, especially as an intermediate compound, for the production of meropenem.

The compound of formula I in crystalline form may be isolated very easily, and has excellent purity and stability. The compound of formula I in crystalline form is therefore very suitable as an intermediate, particularly also on an industrial scale, in the process for producing meropenem. In addition, usage of the compound of formula I in crystalline form has the advantage that other purification steps, such as chromatography, can be dispensed with, since the purity of the compound of formula I in crystalline form is completely sufficient for the production of meropenem.

**Tab. 2: Stability comparisons between compound of formula I in crystalline form and amorphous form**

| | | Compound of formula I in crystalline form | | | compound of formula I in amorphous form | |
|---|---|---|---|---|---|---|
| starting value: | | 99.4% by weight | | | | 92.4% by weight |
| sum of all by-products | | | <0.3% | | | 2.5% |
| | | | | | | |
| 72 h at 70°C | | 99.1 % by weight | | | | 90.8% by weight |
| | sum of all by-products <0.3% | | | sum of all by-products 5.7% | | |

These points clearly indicate that the use of the compound of formula I in crystalline form brings with it considerable advantages over the usage of the previously described amorphous forms of the compound of formula I, which may be reacted and employed especially in the production of meropenem.

Therefore, in a further aspect, the present invention relates to a process for the production of meropenem, which is characterised in that it comprises a step of isolating the compound of formula I in crystalline form, which is then further reacted to meropenem, especially by cleaving the p-nitrobenzyl or p-nitrobenzyloxycarbonyl protecting groups from the carboxyl group in position 2 of the carbapenem frame, as well as from the nitrogen atom of the pyrrolidine ring of the side chain. Cleavage may take place analogously to, e.g. exactly as in known processes for the production of meropenem, for example by hydrogenation as described in EP-A-126587.

The following examples are intended to illustrate the present invention All temperatures are indicated in °C, and all purities in HPLC area percentages.

### Example 1: Crystalline (4R,5S,6S)-(p-nitrobenzyl) 3-[[(3S,5S)-1(-p-nitrobenzyloxycarbonyl)-5-(dimethylaminocarbonyl)-3-pyrrolidinyl]thio]-6-[(1R)-1-hydroxyethyl]-4-methyl-7-oxo-1-azabicyclo[3.2.0]hept-2-ene-2-carboxylate

*p*-nitrobenzyl-(1*R*,5*R*,6*S*)-6-[(1*R*)-1-hydroxyethyl]-2-[(diphenylphosphono)oxy]-1-methylcarbapen-2-em-3-carboxylic acid ester (23.5g, *39.5mmols) and (2*S*,4*S*)-2-dimethylaminocarbonyl-4-mercapto-1-(*p*-nitrobenzyloxycarbonyl)-pyrrolidine (14.8g, 41.9mmols) are dissolved in 100 ml of dimethylacetamide and cooled to -10°C. Over the course of 15 mins, 22 ml of diisopropylethyl amine are added dropwise in such a way that the temperature does not exceed -7°C. The mixture is stirred for 1 hour at -10°C. Then 300 ml of cold ethyl acetate are added over 30 mins at a temperature of < -7°C and finally 200 ml of ice water are added at <-5°C over 30 mins.

The aqueous phase is separated and extracted with 150 ml of ethyl acetate. The combined organic phases are extracted twice, each time with a cold mixture of 80 ml of 6% aqueous NaCl solution and 20 ml of 2N hydrochloric acid, and once with 100 ml of phosphate buffer solution pH 7.0.

The organic phase is separated, filtered and the filter washed with 10 ml of ethyl acetate. The filtrate is concentrated to 90 g at 40°C and made up to 110 g with ethyl acetate and stirred for 72 h at 20°C. The product crystallises. In order to complete crystallisation, 35 ml of heptane are added dropwise and the crystal suspension is stirred for 30 mins. The crystalline product is isolated by filtration, washed twice, each time with 50 ml of heptane, and dried for 16 h at 40°C in a vacuum.
Weighed product: 25.45g (36.5mmols)
purity (HPLC): 98.6%
Melting point: 156-159°C
IR (Golden gate) cm⁻¹: 1762.6, 1702.6, 1638.4, 1519.3, 1444.0, 1402.4, 1341.2, 1317.0, 1273.4, 1205.6, 1179.2, 1138.2, 1111.9, 1045.4, 1017.1, 985.1, 860.0, 841.1, 767.1, 739.4 ¹H-NMR (d₆-DMSO) δ 8.24 (m, 4H), 7.73 (d, J = 9.00 , 2H), 7.66&7.54 (d, J = 9.0, 2H), 5.47&5.30(ABq, 2H,J=14.1Hz), 5.25-5.04(m,3H), 4.80(m,1H), 4.26(m,1H), 4.15(m,1H), 3.99(m,1H), 3.86(m,1H), 3.61(m,1H), 3.29(m,1H), 3.20(m,1H), 3.03&2.97(2s,3H), 2.87(m,1H), 2.83&2.83(2s,3H), 1.63(m, 1H), 1.17(2t, 6H,J=6.0Hz)

### Example 2: Crystalline (4R,5S,6S)-(p-nitrobenzyl) 3-[[(3S,5S)-1(-p-nitrobenzyloxycarbonyl)-5-(dimethylaminocarbonyl)-3-pyrrolidinyl]thiol-6-[(1R)-1-hydroxyethyl]-4-methyl-7-oxo-1-azabicyclo[3.2.0]hept-2-ene-2-carboxylate

2.5 g of amorphous (4*R*,5*S*,6*S*)-(*p*-nitrobenzyl) 3-[[(3*S*,5*S*)-1(-p-nitrobenzyloxycarbonyl)-5-(dimethylaminocarbonyl)-3-pyrrolidinyl]thio]-6-[(1*R*)-1-hydroxyethyl]-4-methyl-7-oxo-1-azabicyclo[3.2.0]hept-2-ene-2-carboxylate (content ca. 90%, HPLC purity: 97.7 area %) are dissolved in 42 mlof ethyl acetate. The amorphous material rapidly goes into solution. The solution is stirred at 20°C, and after about 10 mins, crystallisation sets in. After 1.5 h, 3.5 ml of cyclohexane are added in order to complete crystallisation. The crystal suspension is stirred for 2.5 h.
The crystalline product is isolated by filtration, washed with 5ml of cyclohexane, and dried for 16 h at 40°C in a vacuum.
Weighed product: 1.96g
HPLC purity: 99.8%
Melting point: 159-161°C
¹H-NMR (d₆-DMSO, 300 MHz): identical to above

### Example 3: Further processing of crystalline compound of formula I into meropenem:

1.0 g of the compound obtained in example 1 is dissolved in 60 ml of tetrahydrofuran and 90 ml of water, mixed with 1.0 g of 10% palladium on activated carbon and hydrogenated at atmospheric pressure for 3 h. Subsequently, 50 ml of ethyl acetate are added. Filtration takes place and the organic phase is separated. The aqueous phase is washed once with ethyl acetate and carefully concentrated to 5 ml. 30 ml of tetrahydrofuran are added dropwise to the aqueous solution, which is then seeded and allowed to crystallise at 0°C. The crystalline precipitate of meropenem is filtered off, washed with tetrahydrofuran and dried in a vacuum.
Weighed product: 0.48g
¹H-nmr (D₂O)?δ 1.21 (d,3H,J=7.1 Hz), 1.29(d, 3H,J=6.3Hz), 1.97(m,1H), 2.99(s,3H), 3.06(s,3H), 3.09(m,1H), 3.38(m,1H), 3.46(m,2H), 3.77(dd,1H,J=11.9&6.2Hz), 4.05(m, 1H), 4.23(m,2H), 4.82(1H, signal is partly under D₂O signal)

## Claims

1. Compound of formula 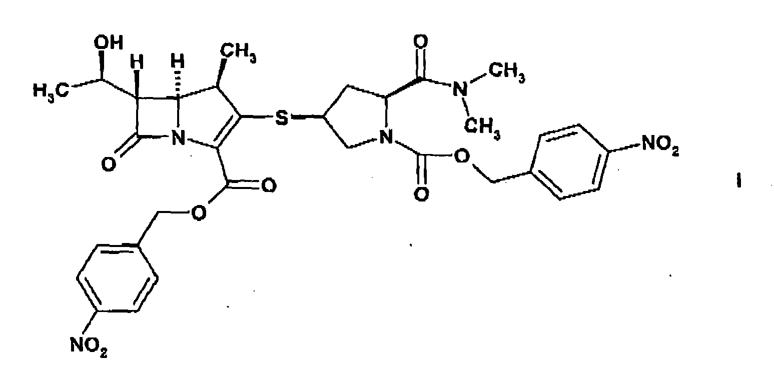 in crystalline form.

2. Compound according to claim 1, which has the following characteristic X-ray diffraction diagram (apparatus used: powder diffractometer AXS-BRUKER D-8, CuK-α radiation, Bragg-Brentano-Optics, scintillation counter, 40kV, 40mA, 0.01° steps, time 2 seconds, cut-off: 40°, standard sample carrier, evaluation was carried out using the software DiffracPlus™):
| angle [2-Theta °] | d value [Ångstrom] | intensity [%] |
|---|---|---|
| 5.091 | 17-34318 | 18.9 |
| 12.815 | 6.90257 | 32.3 |
| 13.181 | 6.71159 | 20.2 |
| 15.010 | 5.89754 | 22.7 |
| 15.480 | 5.71953 | 29.3 |
| 15.848 | 5.58766 | 27.5 |
| 16.348 | 5.41781 | 19.7 |
| 17.608 | 5.03292 | 79.6 |
| 18.367 | 4.82644 | 100.0 |
| 19.758 | 4.48985 | 22.8 |
| 20.316 | 4.36776 | 20.1 |
| 20.602 | 4.30770 | 18.3 |
| 21.133 | 4.20055 | 36.7 |
| 21.477 | 4.13404 | 37.5 |
| 22.120 | 4.01542 | 28.0 |
| 22.404 | 3.96519 | 31.0 |
| 23.411 | 3.79678 | 33.6 |
| 25.872 | 3.44089 | 22.5 |
| 26.605 | 3.34785 | 24.4 |
| 27.545 | 3.23564 | 31.7 |

3. Process for the production of the compound of formula I as defined in claim 1 in crystalline form, comprising the step in which the compound of formula I in non-crystalline form is dissolved in an alkyl alkanoate and crystallisation of the compound of formula I is carried out in order to obtain the compound of formula I in crystalline form.

4. Process according to claim 3, whereby the alkyl alkanoate is a (C₁₋₆)alkylester of acetic acid.

5. Process according to claim 4, whereby the alkyl alkanoate is isopropyl acetate, n-butyl acetate or ethyl acetate.

6. Process according to one of claims 3 to 5, whereby an anti-solvent is added.

7. Process according to claim 6, whereby the anti-solvent is an apolar hydrocarbon.

8. Use of the crystalline compound of formula I as defined in claim 1 in a process for producing meropenem.

9. Process for the production of meropenem comprising the isolation of the compound of formula I, as defined in claim 1, in crystalline form.

10. Process according to claim 9, in which the p-nitrobenzyl and/or p-nitrobenzyloxycarbonyl protecting groups of the compound of formula I, which is isolated in crystalline form, are removed in a further step.

## Patentansprüche

1. Verbindung der Formel 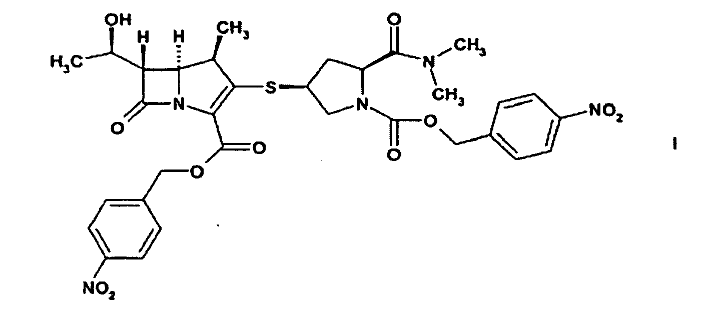 in kristalliner Form.

2. Verbindung nach Anspruch 1, die das folgende charakteristische Röntgenbeugungsdiagramm aufweist (verwendetes Gerät: Pulverdiffraktometer AXS-BRUKER D-8, CuK-α-Strahlung, Bragg-Brentano-Optik, Scintillationszähler, 40 kV, 40 mA, 0,01°-Schritte, Zeit 2 Sekunden, Ausschluss: 40°, Standard-Probenträger, Evaluierung wurde durchgeführt unter Verwendung der Software DiffracPlus™):
| Winkel [2-Theta°] | d-Wert [Ångstrom] | Intensität [%] |
|---|---|---|
| 5,091 | 17,34318 | 18,9 |
| 12,815 | 6,90257 | 32,3 |
| 13,181 | 6,71159 | 20,2 |
| 15,010 | 5,89754 | 22,7 |
| 15,480 | 5,71953 | 29,3 |
| 15,848 | 5,58766 | 27,5 |
| 16,348 | 5,41781 | 19,7 |
| 17,608 | 5,03292 | 79,6 |
| 18,367 | 4,82644 | 100,0 |
| 19,758 | 4,48985 | 22,8 |
| 20,316 | 4,36776 | 20,1 |
| 20,602 | 4,30770 | 18,3 |
| 21,133 | 4,20055 | 36,7 |
| 21,477 | 4,13404 | 37,5 |
| 22,120 | 4,01542 | 28,0 |
| 22,404 | 3,96519 | 31,0 |
| 23,411 | 3,79678 | 33,6 |
| 25,872 | 3,44089 | 22,5 |
| 26,605 | 3,34785 | 24,4 |
| 27,545 | 3,23564 | 31,7 |

3. Verfahren zur Herstellung der wie in Anspruch 1 definierten Verbindung gemäß Formel I in kristalliner Form, umfassend den Schritt, in dem die Verbindung der Formel I in nicht-kristalliner Form in einem Alkylalkanoat gelöst wird und Kristallisation der Verbindung der Formel I durchgeführt wird, um die Verbindung der Formel I in kristalliner Form zu erhalten.

4. Verfahren nach Anspruch 3, wobei das Alkylalkanoat ein (C₁₋₆)Alkylester von Essigsäure ist.

5. Verfahren nach Anspruch 4, wobei das Alkylalkanoat Isopropylacetat, n-Butylacetat oder Ethylacetat ist.

6. Verfahren nach einem der Ansprüche 3 bis 5, wobei ein Antilösungsmittel hinzugefügt wird.

7. Verfahren nach Anspruch 6, wobei das Antilösungsmittel ein apolarer Kohlenwasserstoff ist.

8. Verwendung der wie in Anspruch 1 definierten kristallinen Verbindung von Formel I in einem Verfahren zum Herstellen von Meropenem.

9. Verfahren zum Herstellen von Meropenem, umfassend die Isolierung der wie in Anspruch 1 definierten Verbindung der Formel I in kristalliner Form.

10. Verfahren nach Anspruch 9, in dem die p-Nitrobenzyl- und/oder p-Nitrobenzyloxycarbonylschutzgruppen der Verbindung der Formel I, die in kristalliner Form isoliert wird, in einem weiteren Schritt entfernt werden.

## Revendications

1. Composé de formule 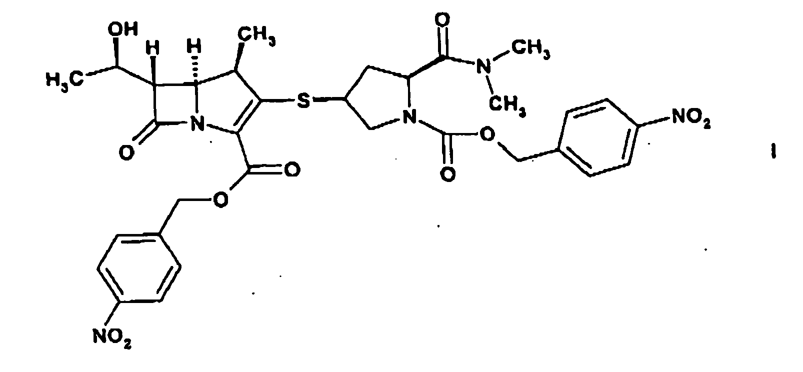 sous forme cristalline.

2. Composé selon la revendication 1, qui a le diagramme caractéristique suivant de diffraction des rayons X (appareil utilisé : diffractomètre sur poudres AXS-BRUKER D-8, rayonnement CuK-α, optique Bragg-Brentano, compteur de scintillation, 40 kV, 40 mA, pas de 0,01°, temps 2 secondes, coupure : 40°, porte-échantillon standard ; l'évaluation a été menée en utilisant le logiciel DiffracPlus™) :
| Angle [2-thêta°] | Valeur d [Angströms] | Intensité [%] |
|---|---|---|
| 5,091 | 17,34318 | 18,9 |
| 12,815 | 6,90257 | 32,3 |
| 13,181 | 6,71159 | 20,2 |
| 15,010 | 5,89754 | 22,7 |
| 15,480 | 5,71963 | 29,3 |
| 15,848 | 5,58766 | 27,5 |
| 16,348 | 5,41781 | 19,7 |
| 17,608 | 5,03292 | 79,6 |
| 18,367 | 4,82644 | 100,0 |
| 19,758 | 4,48985 | 22,8 |
| 20,316 | 4,36776 | 20,1 |
| 20,602 | 4,30770 | 18,3 |
| 21,133 | 4,20055 | 36,7 |
| 21,477 | 4,13404 | 37,5 |
| 22,120 | 4,01542 | 28,0 |
| 22,404 | 3,96519 | 31,0 |
| 23,411 | 3,79678 | 33,6 |
| 25,872 | 3,44089 | 22,5 |
| 26,605 | 3,34785 | 24,4 |
| 27,545 | 3,23564 | 31,7 |

3. Procédé pour la production du composé de formule I selon la revendication 1 sous forme cristalline, comprenant l'étape dans laquelle le composé de formule 1 sous forme non cristalline est dissous dans un alcanoate d'alkyle et une cristallisation du composé de formule I est menée afin d'obtenir le composé de formule I sous forme cristalline.

4. Procédé selon la revendication 3, dans lequel l'alcanoate d'alkyle est un ester alkylique en C₁-C₆ d'acide acétique.

5. Procédé selon la revendication 4, dans lequel l'alcanoate d'alkyle est l'acétate d'isopropyle, l'acétate de n-butyle ou l'acétate d'éthyle.

6. Procédé selon l'une des revendications 3 à 5, dans lequel un anti-solvant est ajouté.

7. Procédé selon la revendication 6, dans lequel l'anti-solvant est un hydrocarbure apolaire.

8. Utilisation du composé cristallin de formule I selon la revendication 1 dans un procédé de production de méropénème.

9. Procédé pour la production de méropénème comprenant l'isolation du composé de formule I selon la revendication 1 sous forme cristalline.

10. Procédé selon la revendication 9, dans lequel les groupes de protection p-nitrobenzyle et/ou p-nitrobenzyloxycarbonyle du composé de formule I, qui est isolé sous forme cristalline, sont supprimés dans une étape suivante.
